# EUROPÄISCHE PATENTSCHRIFT

(11) **EP 2 231 012 B1**
(45) Veröffentlichungstag und Bekanntmachung des Hinweises auf die Patenterteilung: **21.09.2016**
(21) Anmeldenummer: 09702667.8
(22) Anmeldetag: 13.01.2009
(51) Int. Cl.: A61B 5/107, A61B 1/227, G01B 11/24

(54) **HOHLRAUMUNTERSUCHUNGSGERÄT**
CAVITY EXAMINATION DEVICE
APPAREIL D'ANALYSE D'ESPACE CREUX

(30) Priorität: 18.01.2008 DE 102008005070; 18.01.2008 US 21958 P
(43) Veröffentlichungstag der Anmeldung: 29.09.2010
(73) Patentinhaber: Sivantos Pte. Ltd., Singapore 139959 (SG)
(72) Erfinder: RASS, Uwe, 90480 Nürnberg (DE); SAUER, Joseph, 96129 Strullendorf (DE)
(74) Vertreter: FDST Patentanwälte
(86) Internationale Anmeldenummer: PCT/EP2009/050303
(87) Internationale Veröffentlichungsnummer: WO 2009/090161

(56) Entgegenhaltungen:
- EP-A- 1 477 102
- WO-A-2007/004083
- FR-A- 1 278 965
- US-A1- 2003 164 952
- US-A1- 2005 137 456

## Beschreibung

Die vorliegende Erfindung betrifft ein verbessertes Hohlraumuntersuchungsgerät. Insbesondere betrifft die Erfindung ein Hohlraumuntersuchungsgerät, welches ein optisches Empfangselement, beispielsweise eine Linse, einen Bildsensor oder eine Kamera, aufweist und ein Einführen in den Hohlraum ermöglicht, wobei das Empfangselement die Hohlraumwandung nicht berührt.

Zur Untersuchung von Hohlräumen sind vielfältige Geräte bekannt. Auf dem Gebiet der Oto-Rhino-Laryngologie wird beispielsweise ein sogenanntes Otoskop verwendet, welches eine trichter- oder kegelförmige Spitze aufweist, um bei der Untersuchung des Hohlraumes (hier: Ohrkanal oder Nasenöffnung), die Hohlraumwandung (hier: Ohrkanalwand oder Nasenwand) nicht zu verletzen und gleichzeitig ein optisches Empfangselement (hier: Linse) so zu führen, daß es die Hohlraumwandung nicht berührt. Neben den klassischen Otoskopen, die ein Mediziner nutzt, um unmittelbar in den Ohr- bzw. Nasenhohlraum zu schauen, sind beispielsweise aus US 5,363,839 Video-Otoskope mit ebenfalls trichter- oder kegelförmiger Spitze bekannt, bei denen eine Kamera Bilder des Untersuchungsgegenstands aufnimmt.

Neben den Otoskopen, die in erster Linie die Untersuchung am Ende des Hohlraums liegender Objekte (z.B. Trommelfell) ermöglichen, sind Geräte bekannt, die eine dreidimensionale Vermessung von Hohlräumen erlauben. EP 1 477 102 A1 beispielsweise offenbart eine Vorrichtung zum Einführen in einen Körperkanal, z.B. einen Gehörgang. Sie weist flexible Vorrichtungsbestandteile auf, z.B. Scheiben, die eine Positionierung zentral im jeweiligen Körperkanal gewährleisten sollen. WO 2007/004083 A1 beschreibt eine Vorrichtung zum Einführen in einen Gehörgang, die einen endständig angeordneten Ballon zum zuverlässigen Positionieren aufweist.

EP 1 797 813 A1 beschreibt ein solches Gerät, das insbesondere auch zur dreidimensionalen Vermessung des Ohrkanals geeignet ist, etwa um eine optimale Anpassung von im Ohr getragenen Hörgeräten (sog. Im-Ohr-Hörgeräte) an das Ohr zu ermöglichen.

Das aus EP 1 797 813 A1 bekannte Gerät ist jedoch relativ komplex. Kostengünstiger wäre es, eine vergleichsweise einfach konstruiertes stiftförmiges Gerät mit integrierter Kamera in den Hohlraum, beispielsweise den Ohrkanal, einzuführen und die beim Einführen und/oder Entfernen aufgenommene Bildsequenz auszuwerten und daraus ein 3D-Modell des Hohlraums zu berechnen.

EP 1 477 102 A offenbart ein solches Hohlraumuntersuchungsgerät mit einer Anzahl von im Wesentlichen senkrecht zur Einführungsrichtung abstehenden, scheibenförmigen Abstandsteilen, die das Gerät gegenüber den Wandungen der Körperöffnung abstützen und zentrieren und biegeelastisch verformt werden können.

Eine derartiges stiftförmiges Gerät weist allerdings Nachteile auf. Einerseits kann der Kontakt des Geräts mit der Hohlraumwandung, beispielsweise dem Ohrkanal, nicht zuverlässig vermieden werden, so daß Verschmutzungen des vordersten optischen Elements oder Verletzungen des Ohrkanals nicht zuverlässig verhindert werden können. Andererseits müssen flexible Hindernisse wie etwa Haare im Ohrkanal vor der Untersuchung aufwendig entfernt werden, damit diese die aufgenommene Bildsequenz nicht stören.

Es ist daher eine Aufgabe der vorliegenden Erfindung, ein verbessertes Hohlraumuntersuchungsgerät anzugeben, insbesondere ein Hohlraumuntersuchungsgerät, welches ein optisches Empfangselement, beispielsweise eine Linse oder eine Kamera, aufweist und ein Einführen in den Hohlraum ermöglicht, wobei das Empfangselement die Hohlraumwandung nicht berührt.

Diese Aufgabe wird gelöst durch ein Hohlraumuntersuchungsgerät, das ein optisches Empfangselement, welches von einer Wandung eines Hohlraums ausgesandtes oder reflektiertes Licht empfängt, und einen Schirm aus flexiblem Material aufweist, der im wesentlichen die Form eines Rotationskörperstumpfes (z.B. Kegelstumpf, Rotationsparaboloidstumpf) sowie eine erste Öffnung und eine zweite Öffnung aufweist, wobei der Querschnitt der zweiten Öffnung größer ist als der Querschnitt der ersten Öffnung, und wobei die zweite Öffnung hinsichtlich Gestalt und Dimension so gewählt ist, dass sie überwiegend an der Wandung des Hohlraums federnd anliegt.

Der Schirm ist mit der ersten Öffnung an einem Körper des Hohlraumuntersuchungsgeräts auf einer in den Hohlraum zuerst eingeführten Seite des Hohlraumuntersuchungsgeräts befestigt und beim Einführen des Hohlraumuntersuchungsgeräts in den Hohlraum in Richtung des Hohlraumuntersuchungsgerätes geklappt sowie bei Bewegung des Hohlraumuntersuchungsgeräts aus dem Hohlraum heraus umklappt, so dass der Schirm beim Entfernen des Hohlraumuntersuchungsgeräts aus dem Hohlraum weg vom Hohlraumuntersuchungsgerät geklappt ist.

Vorzugsweise wird ein aus Silikon gefertigter Schirm genutzt.

In einer bevorzugten Ausgestaltung wird der Schirm so dimensioniert ist, daß der Schirm die Abbildung des optischen Empfangselements nicht beeinträchtigt, d.h. der Schirmrand befindet sich stets außerhalb des durch den Öffnungswinkel des Empfangselements definierten Abbildungsbereichs des Hohlraumuntersuchungsgeräts.

Vorzugsweise kann der Schirm gegen Schirme mit anders gestalteter und/oder dimensionierter zweiter Öffnung ausgetauscht werden, um eine Anpassung des Hohlraumuntersuchungsgeräts an verschiedene Hohlräume zu erreichen.

Zum Einsatz in sich verjüngenden Hohlräumen kann der Schirm geschlitzt sein.

Eine besonders einfache Konstruktion ergibt sich, wenn das Hohlraumuntersuchungsgerät einen zylindrischen Körper aufweist, d.h. das Hohlraumuntersuchungsgerät besteht beispielsweise aus einer stiftförmigen Kamera mit aufgesetztem Schirm.

Ein solches Hohlraumuntersuchungsgerät mit Kamera eignet sich dann insbesondere zum Erfassen einer Bildsequenz, die einem Steuergerät zugeleitet wird, welches aus der Bildsequenz ein 3D-Modell des Hohlraums errechnet. Vorteilhaft erfolgt die Bildaufzeichnung beim Entfernen des Hohlraumuntersuchungsgeräts aus dem Hohlraum, da dann der vom Hohlraumuntersuchungsgerät weg geklappte Schirm einerseits eine Zentrierung der Kamera bzw. der vordersten Kameralinse bewirkt und anderer-Bildaufzeichnung beim Entfernen des Hohlraumuntersuchungsgeräts aus dem Hohlraum, da dann der vom Hohlraumuntersuchungsgerät weg geklappte Schirm einerseits eine Zentrierung der Kamera bzw. der vordersten Kameralinse bewirkt und andererseits flexible Hindernisse an der Wandung des Hohlraums (etwa Haare im Ohrkanal) aus dem Bildbereich fernhält.

Im folgenden werden Ausführungsbeispiele der Erfindung anhand von Zeichnungen näher erläutert. Dabei zeigen:
Fig. 1a ein erfindungsgemäßes Hohlraumuntersuchungsgerät beim Einführen in einen im Schnitt dargestellten Hohlraum;
Fig. 1b ein erfindungsgemäßes Hohlraumuntersuchungsgerät beim Entfernen aus dem im Schnitt dargestellten Hohlraum; und
Fig. 2 ein erfindungsgemäßes Hohlraumuntersuchungsgerät beim Entfernen aus einem im Schnitt dargestellten Hohlraum mit weiteren Details.

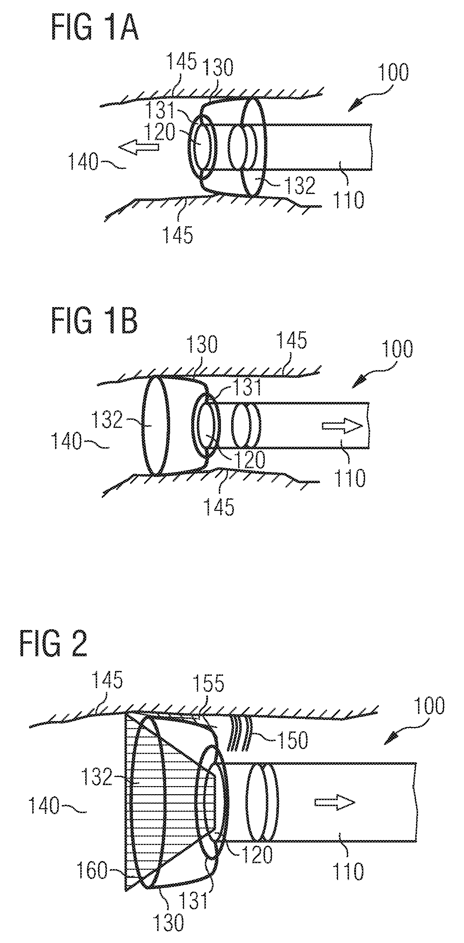

Fig. 1 zeigt ein erfindungsgemäßes Hohlraumuntersuchungsgerät 100, welches einen Gerätekörper 110, ein optisches Empfangselement 120 und einen Schirm 130 aufweist. Das Gerät 100 ist teilweise in einen im Schnitt dargestellten Hohlraum 140 eingeführt, der Wandungen 145 aufweist. Beim Hohlraum 140 kann es sich zum Beispiel um einen menschlichen Ohrkanal handeln, der von den Ohrkanalwänden begrenzt wird. Das optische Empfangselement 120 empfängt von der Wandung 145 des Hohlraums 140 ausgesandtes oder reflektiertes Licht und verarbeitet dieses entweder direkt, falls es sich beim Empfangselement 120 um einen Bildsensor handelt, oder leitet es über weitere optische Elemente weiter, beispielsweise zum Auge eines Betrachters oder zu einem Bildsensor einer Kamera.

Hohlraum 140 ist zumindest auf einer Seite offen; in der Darstellung der Fig. 1 ist dies die Seite, von der her das Hohlraumuntersuchungsgerät 100 teilweise in den Hohlraum 140 eingeführt ist.

In vielen Fällen wird der Hohlraum 140 auf der anderen Seite abgeschlossen sein; im Falle des menschlichen Ohres beispielsweise durch das Körperinnere und/oder das Trommelfell. Um in solchen Fällen mit dem Empfangselement 120 Licht empfangen zu können, ist es häufig notwendig, mittels des Hohlraumuntersuchungsgeräts 100 Licht in den Hohlraum einzuspeisen. Entsprechende Lichtquellen (nicht dargestellt) und deren Integration in das Hohlraumuntersuchungsgerät sind in der Technik wohlbekannt und werden daher hier nicht gesondert behandelt.

Schirm 130 besteht aus einem elastischen Material, beispielsweise Silikon, und ist vorzugsweise ausgebildet als Stumpf eines Rotationskörpers, beispielsweise als Stumpf eines Kegels oder Rotationsparaboloiden, kann allerdings für spezielle Anwendungen auch ein Pyramidenstumpf oder ein beliebiger Hohlkörper sein. Wichtig ist lediglich, daß der Schirm 130 zwei Öffnungen 131, 132 aufweist, wobei die erste Öffnung 131 kleiner ist als die zweite Öffnung 132. Zudem ist die erste Öffnung 131 hinsichtlich Gestaltung und Dimension an den Körper 110 des Hohlraumuntersuchungsgeräts 100 angepaßt, um dort befestigt werden zu können. Bei einem zylindrischen Körper 110 wird die erste Öffnung 131 annähernd ein Kreis passenden Durchmessers sein; bei einem Körper 110 mit rechteckigem Querschnitt hingegen ein Rechteck mit passenden Kantenlängen.

Die zweite Öffnung 132 hingegen ist in Gestaltung und Dimension an den zu untersuchenden Hohlraum 140 angepaßt, und zwar so, daß überwiegend ein federndes Anliegen entlang des Umfangs der Hohlraumwandung 145 gewährleistet ist.

Diese spezielle Dimensionierung des Schirms 130 führt dazu, daß beim Einführen des Hohlraumuntersuchungsgeräts 100 in den Hohlraum 140, wie in Fig. 1a dargestellt, der Schirmrand an der zweiten Öffnung zum Körper 110 des Geräts 100 hin geklappt ist, d.h. weg vom Inneren des Hohlraums 140, und bei Bewegungsumkehr, wie in Fig. 1b dargestellt, umklappt und nunmehr von vom Körper 110 des Geräts 100 weg geklappt ist, d.h. hin vom Inneren des Hohlraums 140.

Dies hat zunächst den Vorteil, daß - anders als bei den bekannten trichter- oder kegelförmigen Spitzen der US 5,363,839 - das optische Empfangselement 120 des Körpers 110 im wesentlichen in der Mitte des zu untersuchenden Hohlraums 140 geführt wird und diese Führung durch die Verwendung des elastischen Materials zu keinen Verletzungen der Wandung führen kann und dort auch nur minimalen Druck ausübt.

Ein weiterer Vorteil der erfindungsgemäßen Schirmanordnung wird im Zusammenhang mit Fig. 2 im folgenden erläutert. Die Darstellung der Fig. 2 entspricht der der Fig. 1b und ist ergänzt um (flexible) Fremdkörper 150, 155, die sich im Hohlraum 140 befinden können, beispielsweise an der Ohrkanalwand wachsende Haare. Diese werden durch den Schirm einfach beiseite gedrückt, dargestellt als verdrängte Haare 155 und stören somit einen schraffiert gezeichneten Bildaufnahmebereich 160 nicht. Vorteilhaft müssen diese Haare nicht vor der Untersuchung durch Rasieren mit speziellen Geräten aufwendig entfernt werden.

Vorteilhaft werden die Parameter des Hohlraumuntersuchungsgeräts 100 so gewählt, daß der Schirm nicht seinerseits die Bildaufnahme behindert. Als wählbare Parameter kommen dabei insbesondere in Betracht: Öffnungswinkel und/oder Brennweite des optischen Empfangselements 120; Querschnittsform und Querschnittsgestalt der zweiten Öffnung 132 sowie die Ausdehnung des Schirms 130 in Richtung von der ersten Öffnung 131 zur zweiten Öffnung 132 hin (d.h. Höhe des bevorzugt verwendeten Rotationskörperstumpfes).

In einem Ausführungsbeispiel weist der Schirm 130 von der zweiten Öffnung her einen Schlitz auf, um sich besser an variierende Querschnitte des zu untersuchenden Hohlraums 140 anpassen zu können.

In einer Ausgestaltung des erfindungsgemäßen Hohlraumuntersuchungsgeräts für die medizinische Anwendung bei der Untersuchung von Ohrkanälen ist es vorteilhaft bzw. sogar notwendig, austauschbare Schirme vorzusehen. Einerseits kann dadurch ein Gerät 100 nach Gebrauch durch Aufsetzen eines frischen Schirms rasch für den nächsten Patienten vorbereitet werden, und andererseits ist es möglich, verschieden großen Ohrkanaldurchmessern durch Verwendung eines entsprechend dimensionierten Schirmes Rechnung zu tragen.

Die 3D-Vermessung eines Ohrkanals, oder allgemeiner eines Hohlraums 140, kann mit dem erfindungsgemäßen Hohlraumuntersuchungsgerät 100 erfolgen, indem das Gerät zunächst soweit wie erforderlich in den Hohlraum 140 eingeführt wird. Anschließend wird die Bildaufnahme gestartet und das Gerät mit geeigneter Geschwindigkeit wieder aus dem Hohlraum entfernt. Der entweder unmittelbar als Empfangselement 120 eingesetzte oder hinter einer als Empfangselement fungierenden Linse angeordnete Bildsensor (nicht dargstellt) leitet dabei die aufgenommenen Bilder an ein Auswertegerät (nicht dargstellt) weiter, welches die Bildersequenz speichert und in Echtzeit oder verzögert ein 3D-Modell des Hohlraums berechnet.

Die optische Vermessung des Ohrkanals wird dabei nicht durch Haare u.ä. gestört. Das optische Empfangselement 120 ist stets nahezu zentriert, und die Ohrkanalwand kommt nur mit dem weichen Silikonschirm in Berührung, nicht jedoch mit dem harten Körper 110.

Es sei darauf hingewiesen, daß die vorliegende Erfindung nicht auf Hohlraumuntersuchungsgeräte beschränkt ist, die ein 3D-Modell des Hohlraums erstellen, sondern beispielsweise auch für (Video-)Otoskope oder kombinierte Meß- und Otoskopie-Untersuchungsgeräte anwendbar ist.

Natürlich kann als flexibles Material außer Silikon auch ein beliebiges anderes flexibles Material wie z.B. Gummi, Naturkautschuk o.ä. verwendet werden.

Ferner ist denkbar, neben starren Gerätekörpern 110 auch biegsame Gerätekörper im Zusammenhand mit der vorliegenden Erfindung zu nutzen, um beispielsweise gebogene Kanäle untersuchen zu können.

## Patentansprüche

1. Hohlraumuntersuchungsgerät (100) mit einem optischen Empfangselement (120), welches von einer Wandung (145) eines Hohlraums (140) ausgesandtes oder reflektiertes Licht empfangen kann , und mit einem Schirm (130) in Form eines Rotationskörperstumpfes aus flexiblem Material,
- wobei der Schirm (130) eine erste Öffnung (131) und eine zweite Öffnung (132) aufweist, deren Querschnitt größer ist als der Querschnitt der ersten Öffnung (131),
- wobei der Schirm (130) mit der ersten Öffnung (131) an einem Körper (120) des Hohlraumuntersuchungsgeräts (100) auf einer in den Hohlraum (140) zuerst einzuführenden Seite des Hohlraumuntersuchungsgeräts (100) befestigt ist, und
- wobei der Schirm (130) derart ausgebildet ist, dass er wenn er beim Einführen des Hohlraumuntersuchungsgeräts (100) in den Hohlraum (140) in Richtung des Hohlraumuntersuchungsgerätes (100) geklappt ist und die zweite Öffnung (132) an der Wandung (145) des Hohlraums (140) federnd anliegt, bei Bewegung des Hohlraumuntersuchungsgeräts (100) aus dem Hohlraum (140) heraus umklappt, so dass der Schirm (130) beim Entfernen des Hohlraumuntersuchungsgeräts (100) aus dem Hohlraum (140) weg vom Hohlraumuntersuchungsgerät (100) geklappt ist.

2. Hohlraumuntersuchungsgerät (100) nach Anspruch 1, dessen Schirm (130) ein Silikonschirm ist.

3. Hohlraumuntersuchungsgerät (100) nach einem der vorhergehenden Ansprüche, dessen Schirm (130) so dimensioniert ist, dass der Schirm (130) eine Abbildung durch das optische Empfangselement (120) nicht beeinträchtigt.

4. Hohlraumuntersuchungsgerät (100) nach einem der vorhergehenden Ansprüche, dessen Schirm (130) austauschbar ist gegen Schirme mit anders gestalteter und/oder dimensionierter zweiter Öffnung, um eine Anpassung des Hohlraumuntersuchungsgeräts (100) an verschiedene Hohlräume zu erreichen.

5. Hohlraumuntersuchungsgerät (100) nach einem der vorhergehenden Ansprüche, dessen Schirm (130) zum Einsatz in sich verjüngenden Hohlräumen geschlitzt ist.

6. Hohlraumuntersuchungsgerät (100) nach einem der vorhergehenden Ansprüche, dessen Körper (120) zylindrisch ist.

7. Hohlraumuntersuchungsgerät (100) nach einem der vorhergehenden Ansprüche, dessen optisches Empfangselement (120) ein Bildsensor, eine Kamera oder die vorderste Linse einer Kameralinsenanordnung ist, wobei der Bildsensor bzw. die Kamera beim Entfernen des Hohlraumuntersuchungsgeräts (100) aus dem Hohlraum (140) eine Bildsequenz erfaßt und einem Steuergerät zuleitet, welches aus der Bildsequenz ein 3D-Modell des Hohlraums (140) errechnet.

8. Hohlraumuntersuchungsgerät (100) nach einem der vorangehenden Ansprüche, welches für die Untersuchung eines menschlichen oder tierischen Ohrkanals dimensioniert ist.

9. Hohlraumuntersuchungsgerät (100) nach einem der vorhergehenden Ansprüche, dessen Schirm (130) so dimensioniert ist, dass der Schirm (130) bei Bewegung des Hohlraumuntersuchungsgeräts (100) aus dem Hohlraum (140) heraus im Hohlraum (140) vorhandene flexible Fremdkörper 150, 155, insbesondere an einer Ohrkanalwand wachsende Haare, aus einem Abbildungsbereich des optischen Empfangselements (120) fernhält.

## Claims

1. Cavity examination device (100) having an optical reception element (120) which can receive light emitted or reflected by a wall (145) of a cavity (140), and having a screen (130) in the shape of a frustum of a body of rotation made of flexible material,
- wherein the screen (130) has a first opening (131) and a second opening (132), the cross section of the second opening (132) being larger than the cross section of the first opening (131),
- wherein the screen (130) is fastened with the first opening (131) on a body (120) of the cavity examination device (100) on a side of the cavity examination device (100) that is to be inserted first into the cavity (140), and
- wherein the screen (130) is designed in such a way that, when it is folded in the direction of the cavity examination device (100) when the cavity examination device (100) is inserted into the cavity (140) and the second opening (132) bears resiliently on the wall (145) of the cavity (140), it is folded back when the cavity examination device (100) is moved out of the cavity (140), so that the screen (130) is folded away from the cavity examination device (100) when the cavity examination device (100) is removed from the cavity (140).

2. Cavity examination device (100) according to Claim 1, the screen (130) of which is a silicone screen.

3. Cavity examination device (100) according to one of the preceding claims, the screen (130) of which is dimensioned so that the screen (130) does not interfere with an imaging by the optical reception element (120).

4. Cavity examination device (100) according to one of the preceding claims, the screen (130) of which can be replaced by screens with a differently configured and/or dimensioned second opening, in order to achieve adaptation of the cavity examination device (100) to different cavities.

5. Cavity examination device (100) according to one of the preceding claims, the screen (130) of which is slit for use in tapering cavities.

6. Cavity examination device (100) according to one of the preceding claims, the body (120) of which is cylindrical.

7. Cavity examination device (100) according to one of the preceding claims, the optical reception element (120) of which is an image sensor, a camera or the front lens of a camera lens arrangement, the image sensor or the camera recording an image sequence when the cavity examination device (100) is removed from the cavity (140), and feeding it to a control device which computes a 3D model of the cavity (140) from the image sequence.

8. Cavity examination device (100) according to one of the preceding claims, which is dimensioned for examining a human or animal auditory canal.

9. Cavity examination device (100) according to one of the preceding claims, the screen (130) of which is dimensioned so that when the cavity examination device (100) is moved out of the cavity (140), the screen (130) keeps flexible foreign bodies (150, 155) present in the cavity (140), in particular hairs growing on an auditory canal wall, away from an imaging region of the optical reception element (120).

## Revendications

1. Appareil d'analyse d'espace creux (100) comportant un élément récepteur optique (120) qui peut recevoir de la lumière émise ou réfléchie par une paroi (145) d'un espace creux (140), et comportant un écran (130) sous forme d'une souche de corps de rotation en matériau souple,
- dans lequel l'écran (130) présente une première ouverture (131) et une seconde ouverture (132) dont la section transversale est supérieure à la section transversale de la première ouverture (131),
- dans lequel l'écran (130) est fixé par la première ouverture (131) à un corps (120) de l'appareil d'analyse d'espace creux (100) sur un côté devant être introduit en premier dans l'espace creux (140) de l'appareil d'analyse d'espace creux (100), et
- dans lequel l'écran (130) est réalisé de manière à être rabattu dans la direction de l'appareil d'analyse d'espace creux (100) lors de l'introduction de l'appareil d'analyse d'espace creux (100) dans l'espace creux (140) et la seconde ouverture (132) appuie élastiquement sur la paroi (145) de l'espace creux (140), et bascule hors de l'espace creux (140) lors du déplacement de l'appareil d'analyse d'espace creux (100) de manière à ce que l'écran (130) soit rabattu en s'éloignant de l'appareil d'analyse d'espace creux (100) lorsque l'appareil d'analyse d'espace creux (100) est retiré de l'espace creux (140).

2. Appareil d'analyse d'espace creux (100) selon la revendication 1, dont l'écran (130) est un écran en silicium.

3. Appareil d'analyse d'espace creux (100) selon l'une quelconque des revendications précédentes, dont l'écran (130) est dimensionné de manière à ce que l'écran (130) n'affecte pas une formation d'image par l'élément récepteur optique (120).

4. Appareil d'analyse d'espace creux (100) selon l'une quelconque des revendications précédentes, dont l'écran (130) peut être remplacé par un écran ayant une deuxième ouverture configurée et/ou dimensionnée différemment afin d'obtenir une adaptation de l'appareil d'analyse d'espace creux (100) à différents espaces creux.

5. Appareil d'analyse d'espace creux (100) selon l'une quelconque des revendications précédentes, dont l'écran (130) est fendu pour être inséré dans des espaces creux effilés.

6. Appareil d'analyse d'espace creux (100) selon l'une quelconque des revendications précédentes, dont le corps (120) est cylindrique.

7. Appareil d'analyse d'espace creux (100) selon l'une quelconque des revendications précédentes, dont l'élément récepteur optique (120) est un capteur d'image, une caméra ou la lentille frontale d'un système de lentilles de caméra, dans lequel le capteur d'image ou la caméra acquiert une séquence d'images lorsque l'appareil d'analyse d'espace creux (100) est retiré de l'espace creux (140) et les envoie à un appareil de commande qui calcule un modèle 3D de l'espace creux (140) à partir de la séquence d'images.

8. Appareil d'analyse d'espace creux (100) selon l'une quelconque des revendications précédentes, qui est dimensionné pour l'analyse d'un canal auditif humain ou animal.

9. Appareil d'analyse d'espace creux (100) selon l'une quelconque des revendications précédentes, dont l'écran (130) est dimensionné de manière à ce que, lors d'un déplacement de l'appareil d'analyse d'espace creux (100) en dehors de l'espace creux (140), l'écran (130) écarte des corps étrangers souples (150, 155) présents dans l'espace creux (140), notamment des poils se développant sur la paroi du canal auditif, hors d'une zone de formation d'image de l'élément récepteur optique (120).
